# EUROPÄISCHE PATENTSCHRIFT

(11) **EP 1 331 962 B1**
(45) Veröffentlichungstag und Bekanntmachung des Hinweises auf die Patenterteilung: **10.01.2007**
(21) Anmeldenummer: 01993488.4
(22) Anmeldetag: 03.11.2001
(51) Int. Cl.: A61M 25/02

(54) **ADAPTER FÜR EINE PEG-SONDE**
ADAPTER FOR A PEG PROBE
ADAPTATEUR POUR UNE SONDE PEG

(30) Priorität: 08.11.2000 DE 10055281
(43) Veröffentlichungstag der Anmeldung: 06.08.2003
(73) Patentinhaber: Fresenius Kabi Deutschland GmbH, 61352 Bad Homburg v.d.H. (DE)
(72) Erfinder: KESSLER, Barbara, 61476 Kronberg (DE); SCHUMACHER, Markus, 52076 Aachen (DE); BREUER-THAL, Barbara, 65795 Hattersheim (DE); KRÜTTEN, Viktor, 65510 Idstein (DE)
(74) Vertreter: Luderschmidt, Schüler & Partner
(86) Internationale Anmeldenummer: PCT/EP2001/012754
(87) Internationale Veröffentlichungsnummer: WO 2002/038212

(56) Entgegenhaltungen:
- WO-A-00/48658
- WO-A-99/25414
- WO-A-99/55409
- US-A- 5 314 411
- US-A- 5 947 931

## Beschreibung

Die Erfindung betrifft einen Adapter zur nachträglichen Verkürzung einer PEG-Sonde, die zur künstlichen Ernährung bereits gelegt worden ist.

Zum Legen einer PEG-Sonde wird ein Endoskop oder Gastroskop in den Magen des Patienten eingeführt, und der Magen wird durch Luftinsuflation entfaltet. Anschließend wird eine Kanüle durch die Bauchdecke und die Magenwand in das Magenlumen vorgeschoben. Durch die Kanüle wird ein Führungsfaden in das Magenlumen eingeführt, mit dem Endoskop oder Gastroskop erfaßt und durch Speiseröhre und Mund des Patienten wieder herausgezogen. Mit Hilfe des auf diese Weise gelegten Führungsfadens wird die Sonde dann bis zum Mageninneren und von dort über die Kanüle nach außen geführt. Dieser Eingriff wird auch als perkutane endoskopisch kontrollierte Gastrostomie (PEG) bezeichnet.

Die bekannten PEG-Sonden, die allgemein Verwendung fmden, weisen an ihrem distalen Ende ein inneres Rückhalteglied auf, mit dem sich der Sondenschlauch an der Mageninnenwandung abstützt. Der Sondenschlauch ist derart bemessen, daß er sich weit aus der Bauchdecke heraus erstreckt. An seinem proximalen Ende weist der Schlauch ein Anschlußteil auf, um das Überleitsystem zum Zuführen von Nährlösung konnektieren zu können. Zum Verschließen des Sondenschlauchs ist häufig noch eine konventionelle Schlauchklemme oder integrierte Verschlußkappe vorgesehen.

In der Praxis haben sich die bekannten PEG-Sonden bewährt. Als störend wird aber vom aktiven Patienten der relativ weit vorstehende Sondenschlauch empfunden.

Die US-A-5,549,657 beschreibt eine PEG-Sonde, die über einen Adapter zum Anschluß eines Überleitsystems verfügt. Der Adapter wird auf den Schlauch der PEG-Sonde aufgeschoben und mittels eines Klemmbügels arretiert. Nachteilig ist, daß der Sondenschlauch unmittelbar oberhalb der Bauchdecke abgeschnitten und exakt auf die richtige Länge gekürzt werden muß. Wird der Sondenschlauch zu kurz abgeschnitten, kann der Adapter nicht mehr befestigt werden, so daß eine neue PEG-Sonde gelegt werden muß. Ist der Sondenschlauch hingegen zu lang, liegt der Adapter nicht auf der Bauchdecke auf. Im übrigen ist die Befestigung des Adapters an dem nur ein kurzes Stück oberhalb der Bauchdecke abgeschnittenen Sondenschlauch relativ schwierig.

Der Adapter wird von einem Schlitzventil verschlossen, das sich beim Konnektieren des Überleitsystems öffnet. Zum Öffnen des Ventils weist der Anschlußteil des Überleitsystems eine vorspringende Kanüle auf, die in den Adapter eingeführt wird. Daher ist die bekannte PEG-Sonde mit den konventionellen Überleitsystemen, die einen Luer-Lock-Anschlußteil aufweisen, nicht direkt kompatibel. Darüber hinaus besteht die Gefahr, daß das Y-Schlitzventil nicht vollständig abdichtet und zu schnell seine Funktionalität verliert.

Die DE 197 49 741 C1 beschreibt eine Fixiervorrichtung zur Befestigung einer PEG-Sonde, die über eine Auflageplatte verfügt. Die Auflageplatte weist eine Ausnehmung zum Durchführen der Sonde und ein Klemmteil auf. Zum Verklemmen der Sonde mit dem Klemmteil wird der Sondenschlauch oberhalb der Bauchdecke um 90°C gebogen. Mittel zum Anschluß des Sondenschlauchs der PEG-Sonde sowie eines Überleitungsschlauchs eines Überleitsystems weist die bekannte Fixiervorrichtung nicht auf.

Die WO 00/48658 beschreibt eine PEG-Sonde mit einem sich an der Magenwandung abstützenden inneren Rückhalteglied an ihrem distalen Ende und einem Anschlußstück an ihrem proximalen Ende. Zur Fixierung der Sonde an der Bauchdecke des Patienten ist ein äußeres Rückhalteglied vorgesehen, das eine Öffnung zum Durchführen des Sondenschlauchs aufweist. Der Sondenschlauch erstreckt sich durch das äußere Rückhalteglied. Nachteilig ist der relativ weit aus der Bauchdecke vorstehende Sondenschlauch.

Aus der WO 99/25414, WO 99/55409 und US-A-5 947 931 sind Befestigungssysteme für Sondenschläuche am Körper des Patienten bekannt. Diese Befestigungssysteme sind aber nicht zur Verkürzung des Sondenschlauches einer PEG-Sonde bestimmt.

Der Erfindung liegt die Aufgabe zugrunde, einen einfach zu handhabenden Adapter mit einer geringen Bauhöhe bereitzustellen, der die nachträgliche Verkürzung des Sondenschlauchs einer bereits gelegten PEG-Sonde erlaubt.

Die Lösung dieser Aufgabe erfolgt erfindungsgemäß mit den Merkmalen des Patentanspruchs 1.

Der erfindungsgemäße Adapter erlaubt die Verkürzung des Sondenschlauchs einer bereits gelegten PEG-Sonde, ohne die Notwendigkeit des Sondentauschs bei noch intaktem Katheterschlauch, wobei der Adapter an den oberhalb der Bauchdecke abgeschnittenen Katheterschlauch angeschlossen wird.

Das Rückhalteglied des erfindungsgemäßen Adapters ist als separates Teil ausgebildet, das eine Öffnung zum Durchführen des Sondenschlauchs der PEG-Sonde aufweist. Die Mittel zur Befestigung des proximalen Endes des Sondenschlauchs und die Mittel zum Anschluß eines Überleitungsschlauchs bilden ein Einsatzstück, das in das Rückhalteglied einsetzbar ist.

Zum Anschluß des Adapters wird der Sondenschlauch auf die erforderliche Länge gekürzt, wobei der Sondenschlauch aber nicht unmittelbar oberhalb der Bauchdecke abgeschnitten zu werden braucht. Das vorstehende Schlauchstück wird darauf hin durch die Öffnung des Rückhalteglieds geführt, und das Rückhalteglied wird auf die Bauchdecke aufgelegt. Erst jetzt erfolgt die Konnektion des Sondenschlauchs, die aufgrund der ausreichenden Schlauchlänge relativ einfach ist. Anschließend wird das Einsatzstück in das Rückhalteglied eingesetzt, so daß der Adapter eine nur geringe Bauhöhe hat.

Damit das Einsatzstück in dem Rückhalteglied ausreichend fixiert ist, weist das Rückhalteglied vorzugsweise einen profilierten Aufnahmeabschnitt und das Einsatzstück einen entsprechend profilierten Einsatzabschnitt auf, der in den Aufnahmeabschnitt passend einsetzbar ist. Das Einsatzstück kann in dem Rückhalteglied klemmend fixiert oder auch nur lose eingelegt sein. Liegt das Einsatzstück nur lose in dem Rückhalteglied, so kann die Fixierung durch den Sondenschlauch erfolgen. Eine derartige Fixierung ist im allgemeinen ausreichend, es können aber auch Klemmbügel oder dgl. zur Arretierung des Einsatzstücks vorgesehen sein.

In einer bevorzugten Ausführungsform weist das Rückhalteglied einen Führungskanal für den Sondenschlauch auf, der sich an die Öffnung anschließt und in den Aufnahmeabschnitt übergeht. Wenn der Sondenschlauch durch die Öffnung des Rückhalteglieds geführt und um 90° umgelegt wird, kann ein besonders flaches Profil erzielt werden, obwohl der Schlauch nicht unmittelbar oberhalb der Bauchdecke abgeschnitten wird, da der Führungskanal das überstehende Schlauchstück aufnimmt. Um den Sondenschlauch klemmend an dem Adapter fixieren zu können, umfaßt der Aufnahmeabschnitt oder Führungskanal des Rückhaltegliedes vorzugsweise einen Klemmabschnitt. Der Klemmabschnitt dient nicht nur zur klemmenden Fixierung des Sondenschlauchs, sondern kann das gesamte Einsatzstück sicher in dem Rückhalteglied festhalten. Im übrigen reduziert der Führungskanal die Knickgefahr des Sondenschlauchs an der Biegestelle.

In einer weiteren bevorzugten Ausführungsform kann das Einsatzstück in unterschiedlichen Positionen in das Rückhalteglied eingesetzt werden, so daß das Rückhalteglied unterschiedliche Schlauchlängen aufnehmen kann. Dies ist insofern von Vorteil, als der Sondenschlauch nicht exakt auf die erforderliche Länge abgeschnitten zu werden braucht.

Das sich an der Bauchdecke abstützende Rückhalteglied des Adadpters sollte aus einem anschmiegsamen, elastischen Material, z. B. Silikonkautschuk mit besonders guten Biokompatibilitätseigenschaften bestehen, während die übrigen Teile des Adapters aus formstabilen Materialien, z. B. thermoplastischen Kunststoffen, gefertigt sein sollten, um dem Adapter die erforderliche Stabilität zu verleihen. Das Rückhalteglied ist vorzugsweise als plattenförmiger Körper ausgebildet, so daß der Adapter eine möglichst geringe Bauhöhe hat.

Zum Verschließen des Adapters ist vorzugsweise ein Absperrorgan mit einem dreh- oder verschiebbaren Verschlußkörper vorgesehen. Das Absperrorgan ist insofern von Vorteil, als das Überleitsystem nicht einen speziellen Anschlußteil aufweisen braucht, der den Adapter beim Konnektieren öffnet. Daher können grundsätzlich alle Applikationssysteme der enteralen Ernährung angeschlossen werden, die über unterschiedliche Anschlußteile verfügen.

Der Verschlußkörper des Absperrorgans kann ein drehbar gelagerter Zylinderkörper sein, der an einem Ende verschlossen, an dem anderen Ende offen und mit einer Querbohrung versehen ist. Wenn der Verschlußkörper das Absperrorgan öffnet, kann Flüssigkeit aus dem Strömungskanal des Adapters durch die Querbohrung in den Zylinderkörper strömen. Dieser Verschlußkörper macht eine Anordnung des Luer-Lock-Anschlußteils quer zum Strömungskanal möglich, wodurch eine besonders geringe Bauhöhe des Adapters erzielt und ein seitlicher Anschluß des Überleitungsschlauchs möglich wird.

Der Verschlußkörper kann aber auch ein Verschlußstück sein, das in den Strömungskanal axial eingeschoben wird. Diese Ausführungsform ist von Vorteil, wenn der Luer-Lock-Anschlußteil in Längsrichtung des Strömungskanals angeordnet sein soll, d. h. der Überleitungsschlauch nicht seitlich angeschlossen wird.

Zur Vereinfachung der Handhabung kann das Verschlußstück durch Drehen des äußeren Gehäusekörpers des Adapters verschoben werden. Die Umwandlung der rotatorischen Bewegung in eine translatorische Bewegung erfolgt dabei vorzugsweise mittels einer Führungsbahn.

Da das Absperrorgan den Adapter dicht verschließt, kann auf eine zusätzliche Verschlußkappe prinzipiell verzichtet werden. Diese ist aber von Vorteil, als sie den Luer-Lock-Aschlußteil des Adapters schützt.

Bei einer bevorzugten Ausführungsform weist der Adapter zum Anschluß des Überleitungsschlauchs einen Luer-Lock-Anschlußteil auf, der sich durch eine geringe Baugröße und sichere Konnexion auszeichnet.

Im folgenden werden verschiedene Ausführungsbeispiele der Erfindung unter Bezugnahme auf die Zeichnungen näher erläutert.

Es zeigen:
- Fig. 1a: eine erste Ausführungsform des an eine PEG-Sonde angeschlossenen Adapters in teilweise geschnittener Darstellung,
- Fig. 1b: den Adapter von Fig. 1a in der Draufsicht,
- Fig. 1c: einen Schnitt durch den Adapter entlang der Linie I-I von Fig. 1a,
- Fig. 1d: eine Explosionsdarstellung des Adapters von Fig. 1a,
- Fig. 2a: ein zweites Ausführungsbeispiel des an die PEG-Sonde angeschlossenen Adapters,
- Fig. 2b: den Adapter von Fig. 2a in der Draufsicht,
- Fig. 2c: einen Schnitt durch den Adapter entlang der Linie II-II von Fig. 2a,
- Fig. 2d: das in das Rückhalteglied einsetzbare Einsatzstück des Adapters von Fig. 2a in vergrößerter, teilweise geschnittener Darstellung, und
- Fig. 3: ein weiteres Ausführungsbeispiel des Adapters in teilweise geschnittener Darstellung, und
- Fig. 4: ein Fixierungselement für den Sondenschlauch.

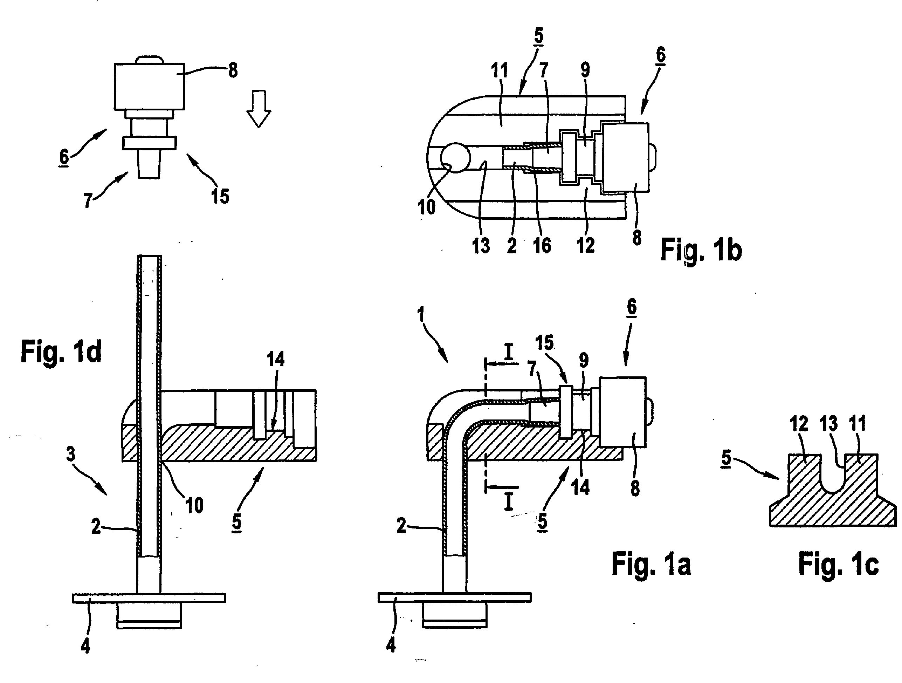

Die Figuren 1a bis 1d zeigen eine erste Ausführungsform des Adapters 1 für den Sondenschlauch 2 einer PEG-Sonde 3. Der Sondenschlauch 2 der PEG-Sonde 3 weist an seinem distalen Ende ein plattenförmiges inneres Rückhalteglied 4 auf, mit dem sich der Schlauch an der Magenwandung abstützt. Das äußere Rückhalteglied 5, mit dem sich die PEG-Sonde an der Bauchdecke abstützt, ist Bestandteil des Adapters.

Der Adapter besteht aus dem plattenförmigen, langgestreckten Rückhalteglied 5, das als separates Teil ausgebildet ist, und einem Einsatzstück 6 mit den Mitteln 7 zur Befestigung des proximalen Endes des Sondenschlauchs 2 und den Mitteln 8 zum Anschluß des Überleitungsschlauchs eines nicht dargestellten Überleitungssystems.

Zum Anschluß des Überleitungsschlauchs weist das Einsatzstück 6 einen positiven Luer-Lock-Anschlußteil 8 auf. An den Luer-Lock-Anschlußteil 8 schließt sich ein zylindrisches Basisteil 9 an, das in seiner Mitte einen verringerten Durchmesser hat. Das zylindrische Basisteil 9 geht in einen konischen Hohlzapfen 7 über, auf den das proximale Ende des Sondenschlauchs 2 aufgeschoben wird.

Das Rückhalteglied 5 weist eine Öffnung 10 zum Durchführen des Sondenschlauchs auf, an die sich ein von zwei parallelen Stegen 11,12 begrenzter Führungskanal 13 für die Schlauchleitung 2 anschließt, der sich in Längsrichtung des Rückhalteglieds erstreckt. Der Führungskanal 13 geht in einen profilierten Aufnahmeabschnitt 14 über, in den ein entsprechend profilierter Einsatzabschnitt 15 des Einsatzstücks 6, der von dem Basisteil 9 und einem Teil des Luer-Lock-Anschlußteils 8 gebildet wird, passend einsetzbar ist.

Der profilierte Aufnahmeabschnitt 14 des Rückhalteglieds 5 weist einen rückwärtigen Klemmabschnitt 16 auf, der derart bemessen ist, daß der auf den Hohlzapfen 7 des Basisteils 9 aufgeschobene Sondenschlauch beim Einsetzen des Einsatzstücks 6 des Adapters klemmend fixiert wird.

Zum Anschluß des Adapters wird der Sondenschlauch 2 durch die Öffnung 10 geführt, auf die richtige Länge abgeschnitten und auf den Hohlzapfen 7 des Einsatzstücks 6 aufgeschoben. Anschließend wird der Sondenschlauch um 90° gebogen und in den Führungskanal eingelegt, und das Einsatzstück wird mit seinem profilierten Einsatzabschnitt 15 in den profilierten Aufnahmeabschnitt 14 des Rückhalteglieds 5 eingesetzt. Zur Reduzierung der Knickgefahr ist das Rückhalteglied im Bereich der Schlauchumlenkung abgerundet. Durch die klemmende Fixierung des Schlauchs in dem Klemmabschnitt wird die Verbindung entlastet. Weiteren Rückhalt bietet die formschlüssige Verbindung von Einsatzstück und Rückhalteglied.

Der nicht dargestellte Überleitungsschlauch des Überleitsystems verfügt über einen negativen Luer-Lock-Anschlußteil. Zum Anschluß des Überleitungsschlauchs werden die beiden Luer-Lock-Anschlußteile des Adapters bzw. des Schlauchs miteinander verbunden.

Die Bereiche des Rückhalteglieds 5 mit direktem Haut- und Gewebekontakt bestehen aus einem anschmiegsamen, biokompatiblen Werkstoff, z. B. Silikonkautschuk, wohingegen die übrigen Teile des Rückhalteglieds aus härteren Materialen, z. B. thermoplastischen Kunststoffen gefertigt sind.

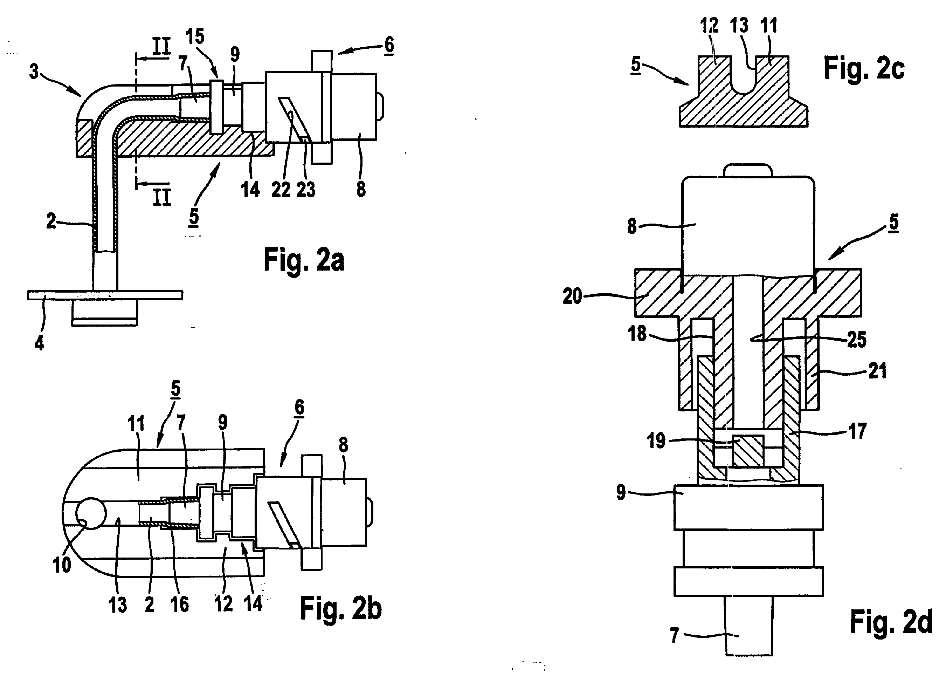

Die Figuren 2a bis 2d zeigen ein zweites Ausführungsbeispiel des Adapters, das sich von der Ausführungsform gemäß der Figuren 1a bis 1 d dadurch unterscheidet, daß der Adapter verschließbar ist. Die Teile des Ausführungsbeispiels gemäß der Figuren 2a bis 2d, die den Teilen der Ausführungsform gemäß der Figuren 1 a bis 1d entsprechen, sind mit den gleichen Bezugszeichen versehen.

Der Adapter verfügt wieder über ein separates Rückhalteglied 5 und ein Einsatzstück 6. Figur 2d zeigt das Einsatzstück dieser Ausführungsform in teilweise geschnittener Darstellung. Das Einsatzstück verfügt über ein Verschlußorgan zwischen dem Luer-Lock-Anschlußteil 8 und dem Basisteil 9. Das Basisteil 9 geht in einen ersten hohlzylindrischen Körper 17 über, in dem ein zweiter hohlzylindrischer Körper 18 längsverschiebbar geführt ist. In dem ersten Zylinderkörper 17 ist ein zylindrisches Verschlußstück 19 konzentrisch angeordnet. Der Luer-Lock-Anschlußteil 8 schließt sich an den zweiten Zylinderkörper 18 an.

An der Unterseite des Luer-Lock-Anschlußteils 8 ist ein ringförmiger Ansatz 20 vorgesehen, von dem sich ein hülsenförmiger Körper 21 erstreckt, der eine Führungsbahn 22 für ein sich von dem ersten Zylinderkörper 17 radial nach außen erstreckenden Führungsstift 23 aufweist (Fig. 2a).

Zum Verschließen der PEG-Sonde 3 wird der obere Teil des Einsatzstückes 6 verdreht, so daß der erste und zweite Zylinderkörper 17, 18 zusammengeschoben werden, wodurch das zylindrische Verschlußstück 9 in den Strömungskanal 25 des zweiten Zylinderkörpers 18 dichtend eingeführt wird. Der ringförmige Ansatz 25 kann mit einer Rändelung oder dgl. versehen sein, um das Einsatzstück 6 besser greifen zu können.

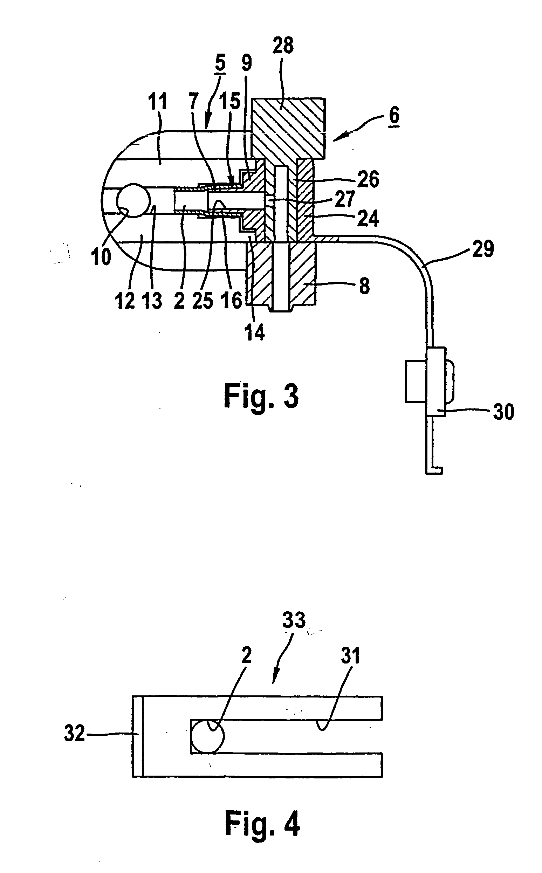

Figur 3 zeigt ein weiteres Ausführungsbeispiel des Adapters in teilweise geschnittener Darstellung. Die einander entsprechenden Teile sind wieder mit den gleichen Bezugszeichen versehen.

Das Einsatzstück 6 weist wieder ein Basisteil 9 mit einem Hohlzapfen 7 zum Aufschieben des Sondenschlauchs 2 auf. An das Basisteil 9 schließt sich der Gehäusekörper 24 des Absperrorgans an, in dem quer zur Längsachse des Strömungskanals 25 in dem Hohlzapfen 7 ein hohlzylindrischer Absperrkörper 26 drehbar gelagert ist, der an einem Ende offen, an dem anderen Ende geschlossen und mit einer zentralen Querbohrung 27 versehen ist. Der Zylinderkörper 26 kann mittels einer Stellschraube 28 gedreht werden, die mit dem Zylinderkörper einstückig ist. Der Luer-Lock-Anschlußteil 8 ist vor dem offenen Ende des Zylinderkörpers 26 an den Gehäusekörper 24 angesetzt. Der Adapter wird durch Drehen des Verschlußkörpers 26 an der Stellschraube 28 geöffnet bzw. geschlossen. An dem Gehäusekörper 24 ist seitlich eine flexible Lasche 29 befestigt, an der eine Verschlußkappe 30 zum Verschließen des Luer-Lock-Anschlußteils 8 angebracht ist.

Das Rückhalteglied 5 unterscheidet sich von dem Rückhalteglied gemäß der Figuren 2a bis 2d nur dadurch, daß der Aufnahmeabschnitt 14 eine dem Einsatzabschnitt 15 des Einsatzstücks 6 entsprechende Profilierung aufweist. Der Aufnahmeabschnitt 14 ist derart profiliert, daß sich das Einsatzstück 6 mit der Stellschraube 28 und dem Luer-Lock-Anschlußteil 8 seitlich an dem Rückhalteglied abstützt. Ansonsten entspricht das Rückhalteglied dem Rückhalteglied gemäß der Figuren 2a bis 2d.

Fig. 4 zeigt ein Fixierungselement 33 für den Sondenschlauch 2 bei der Anlage der PEG-Sonde. Das Fixierungselement ist als flaches Plättchen ausgebildet, das in Längsrichtung eingeschnitten ist. Der Einschnitt 31 hat eine geringere Breite als der Sondenschlauch, so daß der Schlauch mit dem Fixierungselement klemmend fixierbar ist. Die Dicke des Fixierungselements entspricht dem gewünschten Abstand zwischen Bauchdecke und äußerem Rückhalteglied 5 des Adapters. An dem Rand, der dem Einschnitt gegenüberliegt, weist das Fixierungselement einen vorspringenden Ansatz 32 auf. Zur Anlage der PEG-Sonde wird das Fixierungselement auf die Bauchdecke aufgelegt und seitlich auf den Sondenschlauch geschoben, bis der Anschlag 32 an dem Rückhalteglied anschlägt, so daß der Schlauch klemmend fixiert ist. Anschließend wird der Sondenschlauch abgeschnitten, und das Rückhalteglied wird auf den Sondenschlauch geschoben und der Anschlußteil wird befestigt. Dabei dient das Fixierungselement als Abstandshalter.

## Patentansprüche

1. Adapter für eine PEG-Sonde mit einem sich an der Magenwandung abstützenden inneren Rückhalteglied an ihrem distalen Ende und einem offenen proximalen Ende, wobei der Adapter aufweist:
ein sich an der Bauchdecke abstützendes äußeres Rückhalteglied (5), Mittel (7) zur Befestigung des proximalen Endes des Sondenschlauchs der PEG-Sonde und Mittel (8) zum Anschluß eines Überleitungsschlauchs, wobei das Rückhalteglied (5) als separates Teil ausgebildet ist, das eine Öffnung (10) zum Durchführen des Sondenschlauchs (2) aufweist,
**dadurch gekennzeichnet, dass** die Mittel (7) zur Befestigung des proximalen Endes des Sondenschlauchs und die Mittel (8) zum Anschluß des Überleitungsschlauchs ein Einsatzstück (6) bilden, wobei das Rückhalteglied derart ausgebildet ist, dass das Einsatzstück in das Rückhalteglied einsetzbar ist.

2. Adapter nach Anspruch 1,
**dadurch gekennzeichnet, dass** das Rückhalteglied (5) einen profilierten Aufnahmeabschnitt (14) und das Einsatzstück (6) einen entsprechend profilierten Einsatzabschnitt (15) aufweisen, der in den Aufnahmeabschnitt passend einsetzbar ist.

3. Adapter nach Anspruch 1 oder 2,
**dadurch gekennzeichnet, dass** das Rückhalteglied (5) einen Führungskanal (13) für den Sondenschlauch (2) aufweist, der sich an die Öffnung (10) anschließt und in den Aufnahmeabschnitt (14) übergeht.

4. Adapter nach Anspruch 3,
**dadurch gekennzeichnet, dass** der Aumahmeabschnitt (14) des Rückhaltegliedes (5) einen Klemmabschnitt (16) umfaßt, in dem der Sondenschlauch (2) klemmend fixierbar ist.

5. Adapter nach Anspruch 3 oder 4, **dadurch gekennzeichnet, daß** der Aufnahmeabschnitt (14) des Rückhaltegliedes (5) und der Einsatzabschnitt (15) des Einsatzstücks (6) derart profiliert sind, daß das Einsatzstück in unterschiedlichen Positionen in das Rückhalteglied einsetzbar ist.

6. Adapter nach einem der Ansprüche 1 bis 5, **dadurch gekennzeichnet, daß** das Rückhalteglied (5) als plattenförmiger Körper ausgebildet ist.

7. Adapter nach einem der Ansprüche 1 bis 6, **dadurch gekennzeichnet, daß** das Mittel zum Anschluß des Überleitungsschlauchs ein Luer-Lock-Anschlußteil (8) ist.

8. Adapter nach Anspruch 7, **dadurch gekennzeichnet, daß** der Luer-Lock-Anschlußteil (8) mit einer Verschlußkappe (30) verschließbar ist.

9. Adapter nach einem der Ansprüche 1 bis 8, **dadurch gekennzeichnet, daß** ein Absperrorgan mit einem drehbaren oder verschiebbaren Verschlußkörper (19,26) vorgesehen ist, mit dem ein Strömungskanal (25) in dem Adapter verschließbar ist.

10. Adapter nach Anspruch 9, **dadurch gekennzeichnet, daß** der Verschlußkörper ein drehbar gelagerter Zylinderkörper (26) ist, der an einem Ende verschlossen, an dem anderen Ende offen und mit einer Querbohrung (27) versehen ist, wobei das Mittel (8) zur Befestigung des Überleitungsschlauchs an dem offenen Ende des Verschlußkörpers angeordnet ist.

11. Adapter nach Anspruch 9, **dadurch gekennzeichnet, daß** der Verschlußkörper ein zylindrisches Verschlußstück (19) ist, das in den Strömungskanal (25) axial einschiebbar ist.

12. Adapter nach Anspruch 11, **dadurch gekennzeichnet, daß** das zylindrische Verschlußstück (19) in einem ersten Zylinderkörper (17) konzentrisch angeordnet ist, und daß in den ersten Zylinderkörper ein zweiter Zylinderkörper (18) eingesetzt ist, wobei der erste und zweite Zylinderkörper mittels einer Führung (22,23) gegeneinander verschiebbar sind.

13. Adapter nach einem der Ansprüche 1 bis 12, **gekennzeichnet durch** ein Fixierungselement (33) zur klemmenden Fixierung des Sondenschlauchs, das als flaches Plättchen mit einem seitlichen Einschnitt (31) ausgebildet ist.

## Claims

1. An adapter for a PEG probe with an internal retaining element supported on the stomach wall at its distal end and an open proximal end, wherein the adapter comprises:
an external retaining element (5) supported on the abdominal wall, means (7) for the fixing of the proximal end of the probe tube of the PEG probe and means (8) for the connection of a delivery tube, wherein the retaining element (5) is designed as a separate part which has an opening (10) for the passage of the probe tube (2),
**characterised in that** the means (7) for the fixing of the proximal end of the probe tube and the means (8) for the connection of the delivery tube form an insertion piece (6), the retaining element being designed in such a way that the insertion piece can be inserted into the retaining element.

2. The adapter according to claim 1, **characterised in that** the retaining element (5) has a profiled receiving portion (14) and the insertion piece (6) has a correspondingly profiled insertion portion (15) which can be inserted in a matching manner into the receiving portion.

3. The adapter according to claim 1 or 2, **characterised in that** the retaining element (5) has a guide channel (13) for the probe tube (2), which adjoins the opening (10) and transforms into the receiving portion (14).

4. The adapter according to claim 3, **characterised in that** the receiving portion (14) of the retaining element (5) comprises a clamping portion (16), in which the probe tube (2) can be fixed in a clamped manner.

5. The adapter according to claim 3 or 4, **characterised in that** the receiving portion (14) of the retaining element (5) and the insertion portion (15) of the insertion piece (6) are profiled in such a way that the insertion piece can be inserted in different positions into the retaining element.

6. The adapter according to any one of claims 1 to 5, **characterised in that** the retaining element (5) is designed as a plate-shaped body.

7. The adapter according to any one of claims 1 to 6, **characterised in that** the means for the connection of the delivery tube is a Luer lock connector (8).

8. The adapter according to claim 7, **characterised in that** the Luer lock connector (8) can be closed with a closure cap (30).

9. The adapter according to any one of claims 1 to 8, **characterised in that** a shut-off element with a rotatable or displaceable closure body (19, 26) is provided, with which a flow channel (25) in the adapter can be closed.

10. The adapter according to claim 9, **characterised in that** the closure body is a rotatably mounted cylinder body (26) which is closed at one end, open at the other end and provided with a transverse bore (27), wherein the means (8) for the fixing of the delivery tube is disposed at the open end of the closure body.

11. The adapter according to claim 9, **characterised in that** the closure body is a cylindrical closure piece (19), which can be pushed axially into the flow channel (25).

12. The adapter according to claim 11, **characterised in that** the cylindrical closure piece (19) is disposed concentrically in a first cylinder body (17), and that a second cylinder body (18) is inserted into the first cylinder body, wherein the first and second cylinder bodies can be displaced with respect to one another by means of a guide (22, 23).

13. The adapter according to any one of claims 1 to 12, **characterised by** a fixing element (33) for the clamped fixing of the probe tube, said fixing element being designed as a small flat plate with a lateral indent (31).

## Revendications

1. Adaptateur pour une sonde PEG comprenant un élément de retenue intérieur, s'appuyant sur la paroi de l'estomac, sur son extrémité distale et une extrémité proximale ouverte, l'adaptateur présentant :
un élément de retenue (5) extérieur s'appuyant sur la paroi abdominale, des moyens (7) pour la fixation de l'extrémité proximale du flexible de sonde de la sonde PEG et des moyens (8) pour le branchement d'un flexible de transfert, l'élément de retenue (5) étant conçu comme une partie séparée, qui présente une ouverture (10) pour le passage du flexible de sonde (2),
**caractérisé en ce que** les moyens (7) pour la fixation de l'extrémité proximale du flexible de sonde et les moyens (8) pour le raccordement du flexible de transfert forment une pièce d'insertion (6), l'élément de retenue étant conçu de telle sorte que la pièce d'insertion peut être insérée dans l'élément de retenue.

2. Adaptateur selon la revendication 1,
**caractérisé en ce que** l'élément de retenue (5) présente une partie de logement (14) profilée et la pièce d'insertion (6) une partie d'insertion (15) profilée en conséquence, qui peut être insérée de façon adaptée dans la partie de logement.

3. Adaptateur selon la revendication 1 ou 2,
**caractérisé en ce que** l'élément de retenue (5) présente un canal de guidage (13) pour le flexible de sonde (2), qui se raccorde à l'ouverture (10) et fait place à la partie de logement (14).

4. Adaptateur selon la revendication 3,
**caractérisé en ce que** la partie de logement (14) de l'élément de retenue (5) comprend une partie de serrage (16) dans laquelle le flexible de sonde (2) peut être fixé par serrage.

5. Adaptateur selon la revendication 3 ou 4, **caractérisé en ce que** la partie de logement (14) de l'élément de retenue (5) et la partie d'insertion (15) de la pièce d'insertion (6) sont profilées de telle sorte que la pièce d'insertion peut être insérée dans l'élément de retenue dans différentes positions.

6. Adaptateur selon l'une quelconque des revendications 1 à 5, **caractérisé en ce que** l'élément de retenue (5) est conçu comme un corps en forme de plaque.

7. Adaptateur selon l'une quelconque des revendications 1 à 6, **caractérisé en ce que** le moyen pour le raccordement du flexible de transfert est une partie de raccordement de Luer-Lock (8).

8. Adaptateur selon la revendication 7,
**caractérisé en ce que** la partie de raccordement de Luer-Lock (8) peut être fermée avec un capot de fermeture (30).

9. Adaptateur selon l'une quelconque des revendications 1 à 8, **caractérisé en ce qu'**il est prévu un organe d'arrêt avec un corps de fermeture (19, 26) rotatif ou coulissant avec lequel un canal d'écoulement (25) peut être fermé dans l'adaptateur.

10. Adaptateur selon la revendication 9, **caractérisé en ce que** le corps de fermeture est un corps de cylindre (26) logé de façon rotative, qui est fermé sur une extrémité, ouvert sur l'autre extrémité et doté d'un alésage transversal (27), le moyen (8) pour la fixation du flexible de transfert étant disposé sur l'extrémité ouverte du corps de fermeture.

11. Adaptateur selon la revendication 9, **caractérisé en ce que** le corps de fermeture est une pièce d'insertion (19) cylindrique qui peut être introduite axialement dans le canal d'écoulement (25).

12. Adaptateur selon la revendication 11, **caractérisé en ce que** la pièce de fermeture (19) cylindrique est disposée de façon concentrique dans un premier corps de cylindre (17) et **en ce qu'**un second corps de cylindre (18) est inséré dans le premier corps de cylindre, le premier et le second corps de cylindre pouvant coulisser l'un par rapport à l'autre au moyen d'un guide (22, 23).

13. Adaptateur selon l'une quelconque des revendications 1 à 12, **caractérisé par** un élément de fixation (33) pour la fixation par serrage du flexible de sonde, qui est conçu comme une plaquette plate avec une entaille (31) latérale.
